# EUROPEAN PATENT APPLICATION

(11) **EP 4 763 063 A1**
(43) Date of publication of application: **24.06.2026**
(21) Application number: 24902760.8
(22) Date of filing: 09.12.2024
(51) Int. Cl.: A61B 5/0225, A61B 5/022, A61B 5/00, A61B 5/021

(54) **AIRBAG, WRISTBAND, AND WEARABLE ELECTRONIC DEVICE**

(30) Priority: 12.12.2023 CN 202311704599
(71) Applicant: HUAWEI TECHNOLOGIES CO., LTD., Shenzhen 518129 (CN)
(72) Inventor: TIAN, Guoqiang, Shenzhen, Guangdong 518129 (CN); WANG, Youhua, Shenzhen, Guangdong 518129 (CN); JIN, Junye, Shenzhen, Guangdong 518129 (CN); CHENG, Tianyu, Shenzhen, Guangdong 518129 (CN)
(74) Representative: Goddar, Heinz J.
(86) International application number: PCT/CN2024/137792
(87) International publication number: WO 2025/124339

(57) **Abstract**

An airbag (220), a wristband (200), and a wearable electronic device (1000) are disclosed. The airbag (220) includes a first air chamber (221) and a second air chamber (222). The first air chamber (221) and the second air chamber (222) are stacked in a thickness direction of the airbag (220). The first air chamber (221) includes a first main body layer (225) and a second main body layer. The first main body layer (225) and the second main body layer are connected and enclose a first cavity (226). The second air chamber (222) includes a third main body layer (229) and a fourth main body layer. The third main body layer (229) and the fourth main body layer are connected and enclose a second cavity (230). The first cavity (226) communicates with the second cavity (230). The second main body layer and the fourth main body layer are partially connected to form a connection region (231). A region of the second main body layer and a region of the fourth main body layer that are not connected are non-connection regions. A reinforcement member (240) is stacked on the non-connection region. The reinforcement member (240) is adjacent to the connection region (231). The airbag (220) is inflated, the first air chamber (221) and the second air chamber (222) expand in a cavity thickness direction, and the reinforcement member (240) drives the non-connection region of the second main body layer away from the fourth main body layer. The reinforcement member (240) can improve strength of the first air chamber (221) to avoid displacement that affects functions of the first air chamber (221) during inflation.

## Description

This application claims priority to Chinese Patent Application No. 202311704599.2, filed with the China National Intellectual Property Administration on December 12, 2023 and entitled "AIRBAG, WRISTBAND, AND WEARABLE ELECTRONIC DEVICE", which is incorporated herein by reference in its entirety.

### TECHNICAL FIELD

The present invention relates to the field of electronic device technologies, and in particular, to an airbag, a wristband, and a wearable electronic device.

### BACKGROUND

With advancement and development of science and technology, some wearable electronic devices (for example, smart bands and smartwatches) have health monitoring functions such as blood pressure measurement. A smartwatch is used as an example. When measuring blood pressure, a user wears a watchband of an electronic device on a wrist, an airbag on the watchband is inflated and expands, and a wrist artery of the user is compressed by the airbag, so that a pressure sensor located on a hand-contacting side of the airbag extracts a pulse signal of the user, and blood pressure of the user is obtained by calculating the pulse signal. To ensure stability of blood pressure measurement, the airbag needs to have a sufficient width. However, a large width of the airbag causes the user to experience discomfort such as stuffiness when airbag is worn. Therefore, providing a wristband and an airbag that can ensure stability of the blood pressure measurement while having wearing comfort has become a difficult problem to be urgently resolved by a person skilled in the art.

### SUMMARY

This application provides an airbag, a wristband, and a wearable electronic device, to improve stability of airbag measurement while ensuring sufficient wearing comfort of the wearable electronic device.

This application provides an airbag, including a first air chamber and a second air chamber. The first air chamber and the second air chamber are stacked in a thickness direction of the airbag. The first air chamber includes a first main body layer and a second main body layer. The first main body layer and the second main body layer are connected and enclose a first cavity. The second air chamber includes a third main body layer and a fourth main body layer. The third main body layer and the fourth main body layer are connected and enclose a second cavity. The first cavity communicates with the second cavity. The first air chamber and the second air chamber are stacked. The second main body layer and the fourth main body layer are partially connected to form a connection region. A region of the second main body layer and a region of the fourth main body layer that are not connected are non-connection regions. A reinforcement member is stacked on the non-connection region. The reinforcement member is adjacent to the connection region. The airbag is inflated, the first cavity and the second cavity are inflated, the first air chamber and the second air chamber expand in a cavity thickness direction, and the reinforcement member drives the non-connection region of the second main body layer away from the fourth main body layer.

According to a first aspect, in this embodiment of this application, the airbag is disposed on a wristband. The wristband is disposed on a wearable electronic device. The wearable electronic device is worn on a wrist of a user by using the wristband, and the reinforcement member has sufficient strength to support the non-connection region during shape change of the first air chamber, to prevent the non-connection region from shifting from a direction away from the connection region during shape change, that is, avoid displacement and shape change in a width direction of the airbag, prevent the first air chamber from shifting from a gap between the wristband and the wrist, thereby preventing the first air chamber from shifting from a pulse position of the user and affecting measurement accuracy of the wearable electronic device.

In an embodiment, the reinforcement member is provided with a plurality of hollow portions. The plurality of hollow portions are spaced in a length direction of the reinforcement member. Each hollow portion penetrates the reinforcement member in a thickness direction of the first air chamber. The hollow portion can reduce stress of the reinforcement member, and prevent the non-connection region from being wrinkled due to excessive stress of the reinforcement member when the first air chamber expands. The hollow portion is of a through-hole structure, and a shape of the hollow portion is not limited.

In an embodiment, each hollow portion is provided with an opening, and the opening penetrates a side in a width direction of the reinforcement member. The opening may be disposed to improve functionality of the hollow portion and release more stress from the reinforcement member. The opening may face the connection region, or may face away from the connection region. A contour of the opening may be a semicircle, a three-quarter circle, a curved surface, or an arc. A plurality of openings are spaced in the length direction of the reinforcement member to form a curved surface, and the curved surface may be wavy.

In an embodiment, the connection region is provided with a plurality of vent holes. The plurality of vent holes connect the first cavity and the second cavity. The plurality of vent holes are spaced in a length direction of the connection region. In a width direction of the connection region, the plurality of vent holes are in a one-to-one correspondence to the plurality of hollow portions, the opening faces the vent hole, and a middle region of the vent holes is opposite to the opening of the hollow portion. During expansion of the first air chamber, the second main body layer changes in shape, the vent hole changes in shapes, a connection region around the vent hole changes in shape in the width direction of the connection region (a width direction of the first air chamber), and the opening provides shape change space for shape change of the vent hole.

In an embodiment, hardness of the reinforcement member is greater than hardness of the first air chamber, to implement strength support for the non-connection region and avoid shifting of the non-connection region.

In an embodiment, in the width direction of the first air chamber, a width of the reinforcement member is less than or equal to a width of a first support portion, to ensure effective reinforcement of the reinforcement member. The width of the reinforcement member is greater than or equal to 1 mm and less than or equal to 7 mm.

In an embodiment, spacings between every two hollow portions are the same, to improve stress uniformity of the reinforcement member, thereby ensuring uniformity of supporting the first air chamber.

In an embodiment, the connection region is provided with a plurality of vent holes. The plurality of vent holes connect the first cavity and the second cavity. The connection region is divided into a first part and a second part. At least a part of the plurality of vent holes are located in the first part, or a part of the plurality of vent holes are located in the first part, and the other part is located in the second part. A quantity of vent holes in the first part is the same as a quantity of vent holes in the second part. A cross-sectional area of the vent hole in the first part is greater than a cross-sectional area of the vent hole in the second part. It may be understood that, a ventilation area of a first sub-hole is greater than a ventilation area of a second sub-hole. A pulse signal includes a radial artery pulse signal and an ulnar artery pulse signal. The first sub-hole corresponds to a region of a radial artery. The second sub-hole corresponds to a region of an ulnar artery. In a process in which air flows from the second cavity to the first cavity when the airbag is inflated, a ventilation speed at which air passes through the first sub-hole is higher than a ventilation speed at which air passes through the second sub-hole, so that a region that is of the first air chamber and that corresponds to the first sub-hole has a larger attachment surface with the wrist, and the ulnar artery pulse signal can be filtered out while the radial artery pulse signal is accurately detected, to obtain a desired pulse signal.

In an embodiment, two end parts of the reinforcement member in a length direction of the first air chamber have a chamfer contour or a semi-circular arc contour.

In an embodiment, the reinforcement member is fastened to the second main body layer by using a pressing process. The reinforcement member is fastened to a first support portion in a hot-pressing manner, to implement integration of the reinforcement member and the first support portion, improve connection stability, and ensure that the first support portion can change in shape.

In an embodiment, the airbag includes a third air chamber. The third air chamber is stacked between the first air chamber and the second air chamber. The third air chamber includes a fifth main body layer and a sixth main body layer. The fifth main body layer and the sixth main body layer are respectively partially connected to the second main body layer and the fourth main body layer. The fifth main body layer and the sixth main body layer have non-connection regions. The reinforcement member is disposed in the non-connection region of the fifth main body layer, and/or the reinforcement member is disposed in the non-connection region of the sixth main body layer.

In an embodiment, the airbag includes a sensor, an air nozzle, and a wire. The sensor is disposed in the first cavity. The air nozzle is disposed at one end of the second air chamber and connects the outside and the second cavity. The wire is connected to the sensor and extends out from the air nozzle through the first cavity, a vent hole, and the second cavity. The wire extends out from the air nozzle without requiring an additional opening on the airbag. This ensures sealing performance of the airbag and test performance.

According to a second aspect, an embodiment provides a wristband, including a band body and an airbag. The airbag is disposed on a side of the band body, and a second air chamber is connected to the band body. A reinforcement member has sufficient strength to support a first air chamber shape change of the first air chamber, to avoid shifting of the first air chamber during shape change, and ensure stability of a relative position between the airbag and human body.

In an embodiment, the band body and the airbag are of an integrated structure. The integrated structure of the band body and the airbag can improve stability of connection between the band body and the airbag in a process of inflation and deflation.

According to a third aspect, an embodiment provides a wearable electronic device, including a body and a wristband. The wristband is connected to two opposite ends in a length direction of the body. An airbag is disposed on an inner side of the wristband. A structure design of a double-layer airbag of the wearable electronic device provided in this application ensures that the airbag in an inflated state generates sufficient pressure on a wrist of a user, to improve accuracy of test data.

### BRIEF DESCRIPTION OF DRAWINGS

To describe the technical solutions in this application more clearly, the following briefly describes the accompanying drawings for describing implementations. Clearly, the accompanying drawings in the following description show merely some implementations of this application, and a person of ordinary skill in the art may still derive other drawings from these accompanying drawings without creative efforts.
FIG. 1 is a diagram of a structure of a wearable electronic device according to an embodiment of this application;
FIG. 2 is a cross-sectional view of a structure of a first wristband in FIG. 1;
FIG. 3 is a diagram of a structure of an airbag shown in FIG. 1;
FIG. 4 is an exploded view of a structure of the airbag shown in FIG. 3;
FIG. 5 is an exploded view of the airbag shown in FIG. 3 from another perspective;
FIG. 6 is a cross-sectional view of a structure of the airbag shown in FIG. 3;
FIG. 7 is a diagram of a structure of a reinforcement member shown in FIG. 3;
FIG. 8a is a simplified cross-sectional view of a first air chamber in FIG. 2 according to an embodiment;
FIG. 8b is a simplified cross-sectional view of a first air chamber in FIG. 2 according to a second embodiment;
FIG. 8c is a simplified cross-sectional view of a first air chamber in FIG. 2 according to a third embodiment;
FIG. 8d is a simplified cross-sectional view of a first air chamber in FIG. 2 according to a fourth embodiment;
FIG. 8e is a simplified cross-sectional view of a first air chamber in FIG. 2 according to a fifth embodiment;
FIG. 8f is a simplified cross-sectional view of a first air chamber in FIG. 2 according to a sixth embodiment;
FIG. 8g is a simplified cross-sectional view of a first air chamber in FIG. 2 according to a seventh embodiment;
FIG. 9 is a cross-sectional view of a structure of the airbag shown in FIG. 3 from another perspective;
FIG. 10a is a simplified diagram of a structure of an airbag in FIG. 2 according to an embodiment; and
FIG. 10b is a simplified diagram of a structure of an airbag in FIG. 2 according to another embodiment.

Names corresponding reference numerals in the accompanying drawings are as follows: 1000: electronic device; 100: body; 200: wristband; 200a: first wristband; 200b: second wristband; 110: display; 120: rear housing; 130: middle frame; 210: band body; 220: airbag; 221: first air chamber; 222: second air chamber; 223: first connection portion; 2231: first vent hole; 224: first support portion; 225: first main body layer; 226: first cavity; 227: second connection portion; 2271: second vent hole; 228: second support portion; 229: third main body layer; 230: second cavity; 231: connection region; 232: vent hole; 232a: first sub-hole; 232b: second sub-hole; 240: reinforcement member; 241: hollow portion; 250: sensor; 251: wire; 260: air nozzle; 270: attachment region; 280: locking portion; 281: buckle; 282: buckle tongue; 283: buckle hole.

### DESCRIPTION OF EMBODIMENTS

The following clearly and completely describes the technical solutions in embodiments of this application with reference to the accompanying drawings in embodiments of this application. It is clear that the described embodiments are merely some rather than all of embodiments of this application. All other embodiments obtained by a person of ordinary skill in the art based on embodiments of this application without creative efforts shall fall within the protection scope of this application.

For ease of understanding, terms in embodiments of this application are first explained.

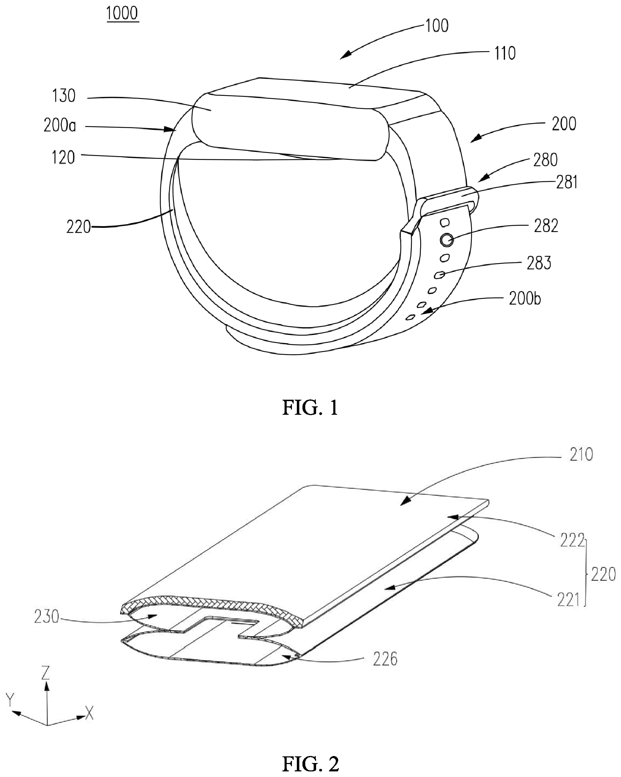

Refer to FIG. 1. FIG. 1 is a diagram of a structure of a wearable electronic device according to an embodiment of this application. The wearable electronic device 1000 may be, but is not limited to, a wearable electronic device such as a watch or a band, and may be used in fields such as daily wearable monitoring devices and medical devices. The wearable electronic device 1000 may be worn on a wrist of a user, and can measure blood pressure of the user at any time and monitor a physical status of the user. In this embodiment of this application, a smartwatch is used as an example of the wearable electronic device 1000 for description. A basic principle of measuring blood pressure by the smartwatch is as follows: When the user uses the smartwatch to measure blood pressure, the smartwatch is worn on the wrist of the user, and both a wristband of the smartwatch and an airbag on the wristband encircle the wrist of the user, and cover an ulnar artery and a radial artery of the user. An air nozzle is driven to inflate the airbag through a UI control interface on the smartwatch. The airbag is pressurized and expands, and compresses the artery. A sensor located on a hand-contacting side of the airbag collects a pulse signal from a wrist artery of the user, and obtains blood pressure of the user by using a related processing module.

For ease of description, in this application, a length direction of the wearable electronic device 1000 is defined as an X-axis direction, a width direction of the wearable electronic device 1000 is defined as a Y-axis direction, and a height direction of the wearable electronic device 1000 is defined as a Z-axis direction. The X-axis, Y-axis, and Z-axis directions are perpendicular to each other. It should be noted that orientation terms such as "up" and "down" in this application are described with reference to orientation shown in FIG. 1. A positive direction toward the Z-axis is "up", and a negative direction toward the Z-axis is "down". This does not indicate or imply that an indicated apparatus or element shall have a specific orientation, or be constructed and operated in a specific orientation. Therefore, this cannot be understood as a limitation on this application.

The wearable electronic device 1000 includes a body 100 and a wristband 200. The wristband 200 is connected to two opposite ends of the body 100. When the user wears the wearable electronic device 1000, the wristband 200 encircles the wrist of the user. The wristband 200 may be of an integrated structure, for example, a band. The wristband 200 may alternatively be divided into two parts. In this embodiment, the wristband 200 is divided into two parts: a first wristband 200a and a second wristband 200b. The first wristband 200a and the second wristband 200b may have a same structure or different structures. The first wristband 200a and the second wristband 200b are connected to the two opposite ends in a length direction of the body 100 (an X direction shown in the figure). Specifically, a first clamping portion (not shown in the figure) is disposed at the two opposite ends in the length direction of the body 100, and is used to connect the body 100 to the first wristband 200a and the second wristband 200b. When the user wears the wearable electronic device 1000, the first wristband 200a and the second wristband 200b encircle the wrist of the user and are fastened to each other. The wristband 200 may be made of a plastic material, a belt, or woven fiber.

The body 100 includes a display 110, a rear housing 120, a middle frame 130, a processor (not shown in the figure), a circuit board (not shown in the figure), a power module (not shown in the figure), and the like. The rear housing 120 is mounted on a side of the middle frame 130, and together with the middle frame 130, encloses accommodation space. The display 110 is mounted on the other side of the middle frame 130, disposed opposite to the rear housing 120 in a thickness direction of the middle frame 130, and encloses the accommodation space enclosed by the middle frame 130 and the rear housing 120. The display 110 displays a control interface and blood pressure of the user for the wearable electronic device 1000. The processor, the circuit board, the power module, and the like are mounted in the accommodation space enclosed by the rear housing 120 and the middle frame 130. The power module is electrically connected to the circuit board and supplies power to the wearable electronic device 1000. The processor may be a CPU (central processing unit, central processing unit) of the wearable electronic device 1000. The processor is electrically mounted on the circuit board, and is configured to: process pulse signal data of the user and drive the display 110 to display blood pressure of the user. The display 110 may be disposed on a panel surface of the body 100, or may be used as a panel surface of the entire body 100. The display is configured to control the wearable electronic device 1000 to display content such as incoming call information, news, and function information, and to synchronize a call, an SMS message, a health monitoring structure, a photo, music, and the like in a mobile phone function. The display 110 may be a liquid crystal display, an organic light-emitting diode display, or the like. A functional component for implementing an entertainment function, health monitoring, exercise monitoring, and navigation may be disposed in the body 100, or may be disposed on the wristband 200. The wearable electronic device 1000 further includes a memory, a communications module, an antenna, and the like. The memory, the communications module, and the antenna are electrically connected to the circuit board.

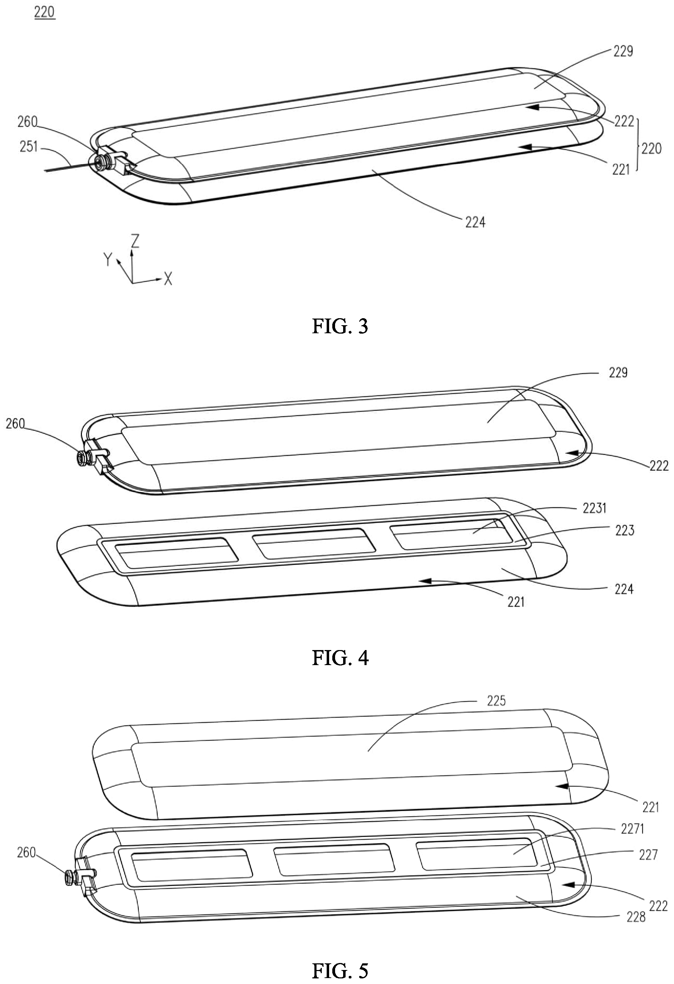

Refer to FIG. 2 and FIG. 3. FIG. 2 is a cross-sectional view of a structure of the first wristband in FIG. 1, and FIG. 3 is a diagram of a structure of an airbag shown in FIG. 1. The first wristband 200a is of a strip structure, and includes a band body 210 and a second clamping portion (not shown in the figure). The band body 210 includes two ends (not shown in the figure) that are disposed opposite to each other, and the two ends are two opposite ends in a length direction of the first wristband 200a. The second clamping portion is disposed at one end of the first wristband 200a, and is configured to be fastened to one first clamping portion (not shown in the figure) of the body 100, for a connection between the first wristband 200a and the body 100. The second wristband 200b is also provided with a second clamping portion (not shown in the figure), and is fastened to another first clamping portion (not shown in the figure) of the body 100.

The wearable electronic device 1000 further includes the airbag 220. The airbag 220 is disposed on the wristband 200. The airbag 220 may automatically inflate or deflate, to implement a blood pressure measurement function. A test result is displayed through the display 110 of the wearable electronic device 1000. The wearable electronic device 1000 is provided with a functional component that can cooperate with the airbag 220. The functional component is configured to: convert a signal detected by the airbag into a pulse signal and read the signal. The airbag 220 may be made of a leather or a flexible material with elasticity and non-breathable. The airbag 220 and the wristband 200 may be fastened through pressing or bonding, but are not limited thereto. In this embodiment, the airbag 220 is fastened to the wristband 200 in a pressing manner. The wristband 200 and the airbag 220 encircles the wrist of the user, and the airbag 220 is in contact with the wrist of the user. In this embodiment, the airbag 220 is disposed on a side of the first wristband 200a. Certainly, the airbag 220 may alternatively be disposed on a side of the second wristband 200b.

Specifically, the airbag 220 is located on a side of the band body 210, and the airbag 220 is fastened to the band body 210 in a pressing manner. It may be understood that the airbag 220 and the band body 210 are of an integrated structure, so that stability of a connection between the band body 210 and the airbag 220 in a process of inflating and deflating can be improved.

Refer to FIG. 4 and FIG. 5. FIG. 4 is an exploded view of a structure of the airbag shown in FIG. 3, and FIG. 5 is an exploded view of the airbag shown in FIG. 3 from another perspective. In this embodiment, the airbag 220 includes a first air chamber 221 and a second air chamber 222. The first air chamber 221 and the second air chamber 222 are stacked and fastened in a thickness direction of the airbag 220. In this embodiment, the airbag 220 is a cuboid. In another embodiment, the airbag 220 may alternatively be a circular shape. The first air chamber 221 is located on a side that is of the second air chamber 222 and that is away from the band body 210. It may be understood that the first air chamber 221 is disposed on a side that is of the first wristband 200a and that is in contact with the wrist of the user.

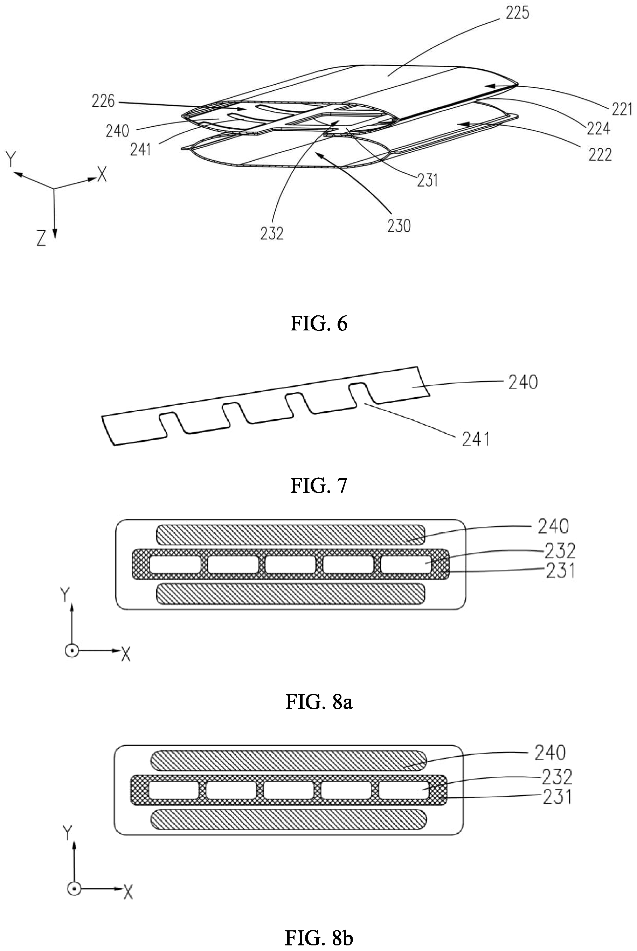

Refer to FIG. 6. FIG. 6 is a cross-sectional view of a structure of the airbag shown in FIG. 3. The first air chamber 221 includes a first connection portion 223, a first support portion 224, and a first main body layer 225. In a circumferential direction of the first air chamber 221, the first support portion 224 connects the first connection portion 223 and the first main body layer 225, and the first support portion 224, the first connection portion 223, and the first main body layer 225 enclose a first cavity 226. The first support portion 224 is located on a circumferential side of the first connection portion 223. The first support portion 224 and the first connection portion 223 are opposite to the first main body layer 225 in the thickness direction of the airbag. It may be understood that the first connection portion 223 and the first support portion 224 are a second main body layer. A circumferential edge of the first main body layer 225 is connected to a circumferential edge of the second main body layer to enclose the first cavity 226. In this embodiment, the first air chamber 221 is made of an elastic material. After the first cavity 226 is inflated, the first air chamber 221 expands. In other words, the first connection portion 223, the first support portion 224, and the first main body layer 225 change in shape. It should be noted that the first connection portion 223, the first support portion 224, and the first main body layer 225 are of an integrated structure. The first connection portion 223 is provided with at least two first vent holes 2231. The first vent hole 2231 penetrates the first connection portion 223 in a thickness direction of the first connection portion 223, and communicates with the first cavity 226. In this embodiment, the first connection portion 223 is provided with a plurality of first vent holes 2231, and the plurality of first vent holes 2231 are spaced part in a length direction of the first air chamber 221. The plurality of first vent holes 2231 have a same shape and a same cross-sectional area. In another embodiment, shapes and cross-sectional areas of the plurality of first vent holes 2231 may be different. A shape of the first vent hole 2231 may be rectangular, circular, or irregular.

A structure of the second air chamber 222 is basically the same as a structure of the first air chamber 221, including a second connection portion 227, a second support portion 228, and a third main body layer 229. In a circumferential direction of the second air chamber 222, the second support portion 228 connects the second connection portion 227 and the third main body layer 229, and the second support portion 228, the second connection portion 227, and the third main body layer 229 enclose a second cavity 230. The second support portion 228 is located on a circumferential side of the second connection portion 227. The second support portion 228 and the second connection portion 227 are opposite to the third main body layer 229 in the thickness direction of the airbag. It may be understood that the second connection portion 227 and the second support portion 228 are a fourth main body layer. A circumferential edge of the third main body layer 229 is connected to a circumferential edge of the fourth main body layer to enclose a second cavity 230. After the second cavity 230 is inflated, the second air chamber 222 expands. In other words, the second connection portion 227, the second support portion 228, and the third main body layer 229 change in shape.

It should be noted that the second connection portion 227, the second support portion 228, and the third main body layer 229 are of an integrated structure. The second connection portion 227 is provided with at least two second vent holes 2271. The second vent hole 2271 penetrates the second connection portion 227 in a thickness direction of the second connection portion 227, and communicates with the second cavity 230. In this embodiment, the second connection portion 227 is provided with a plurality of second vent holes 2271, and the plurality of second vent holes 2271 are spaced in a length direction of the second air chamber 222. The plurality of second vent holes 2271 have a same shape and a same cross-sectional area. In another embodiment, shapes and cross-sectional areas of the plurality of second vent holes 2271 may be different. A shape of the second vent hole 2271 may be rectangular, circular, or irregular.

In the thickness direction of the airbag 220, the first air chamber 221 is connected to the second air chamber 222, and the second main body layer and the fourth main body layer are partially pressed, to form a connection region 231. A plurality of vent holes 232 are provided on the connection region 231. Specifically, the first connection portion 223 of the first air chamber 221 and the second connection portion 227 of the second air chamber 222 are pressed and connected in a hot-pressing manner, to form the connection region 231 of the airbag 220. The first vent hole 2231 and the second vent hole 2271 are in a one-to-one correspondence and communicate with each other to form the plurality of vent holes 232. The vent hole 232 is located in the connection region 231 and connects the first cavity 226 and the second cavity 230. The vent hole 232 penetrates both the first connection portion 223 and the second connection portion 227 in the thickness direction of the first connection portion 223 and the second connection portion 227. The vent hole 232 allows air to flow between the first cavity 226 of the first air chamber 221 and the second cavity 230 of the second air chamber 222 during inflation or deflation of the airbag 220. The connection region 231 is pressed by the first connection portion 223 and the second connection portion 227. In other words, a part of the second main body layer is connected to a part of the fourth main body layer. Hardness of the connection region 231 is greater than that of another region of the airbag 220. This is more conducive to flow of air between the first air chamber 221 and the second air chamber 222.

In this embodiment, a region of the first connection portion 223 located in every two first vent holes 2231 is in contact with and not fastened to a region of the second connection portion 227 located in every two second vent holes 2271, that is, no pressing is performed. This part may be referred to as an adjustment region, and the adjustment region is located in the connection region 231. When air passes through the vent hole to inflate and expand the first air chamber 221 and the second air chamber 222, the adjustment region can improve flexibility of the connection region, and avoid affecting shape change of the connection region. It should be noted that a shape of the vent hole 232 changes during inflation. For example, the vent hole 232 in this embodiment is rectangular, and a middle position of the vent hole 232 expands and changes in shape toward two sides in a width direction.

In the thickness direction of the airbag 220, the first support portion 224 of the first air chamber 221 and the second support portion 228 of the second air chamber 222 are disposed opposite to each other. During inflation of the airbag 220, that is, the first cavity 226 and the second cavity 230 are filled with air, the first main body layer 225 of the first air chamber 221 and the third main body layer 229 of the second air chamber 222 move away from each other, and the first support portion 224 of the first air chamber 221 and the second support portion 228 of the second air chamber 222 are away from each other and are spaced. Actually, both the first air chamber 221 and the second air chamber 222 change in shape and expand. Specifically, during inflation and expansion of the airbag 220, the first air chamber 221 and the second air chamber 222 change in shape, and generate elastic shape change. Certainly, the first air chamber 221 and the second air chamber 222 may alternatively not elastically change in shape. In other words, the elastic shape change is a slight amount of shape change.

The airbag 220 is further provided with an air nozzle 260. In this embodiment, the air nozzle 260 is disposed at one end of the second air chamber 222. The air nozzle 260 and the second air chamber 222 may be integrally formed, or may be additionally mounted on the second air chamber 222. The air nozzle 260 is of a tubular structure, and configured to connect the outside and the second cavity 230 of the second air chamber 222, to inflate and deflate the airbag 220. The air nozzle 260 is located at an end part of the second air chamber 222, and located at a middle position of the end part of the second air chamber 222. The air nozzle 260 corresponds to the second connection portion 227. After the air nozzle 260 is opened, when air enters the second cavity 230 through the air nozzle 260, the air nozzle 260 corresponds to the second vent hole 2271 of the second connection portion 227 in the length direction of the second air chamber 222, and air quickly enters the first air chamber 221 through the second air chamber 222.

Refer to FIG. 7 and FIG. 8a. FIG. 7 is a diagram of a structure of a reinforcement member shown in FIG. 3, and FIG. 8a is a simplified cross-sectional view of the first air chamber in FIG. 2 according to an embodiment. In this embodiment, a difference between the first air chamber 221 and the second air chamber lies in that the first air chamber 221 further includes two reinforcement members 240. The reinforcement member 240 is disposed in the first air chamber 221, and the two reinforcement members 240 are located on two sides in a width direction of the airbag 220. Specifically, the reinforcement member 240 is a bendable strip-shaped sheet, and hardness of the reinforcement member 240 is higher than hardness of the first support portion 224. The reinforcement member 240 is located in the first cavity 226 (an inner surface of the first support portion 224), and is stacked on the first support portion 224 of the first air chamber 221. In another embodiment, the reinforcement member 240 is located on an outer surface of the first support portion 224. In the width direction of the airbag 220, the two reinforcement members 240 are located on two opposite sides of the first connection portion 223, and are spaced. It may also be understood that the two reinforcement members 240 are fastened to the first support portions 224 on the two opposite sides of the first connection portion 223.

The reinforcement member 240 extends in the length direction of the first air chamber 221. An orthographic projection of the reinforcement member 240 onto the first support portion 224 is less than or equal to the first support portion 224. It may be understood that a length of the reinforcement member 240 is less than or equal to a length of the first support portion 224, and a width of the reinforcement member 240 is less than or equal to a width of the first support portion 224. In this embodiment, the reinforcement member 240 is fastened to the first support portion 224 by using a pressing process. In another embodiment, the reinforcement member 240 and the first support portion 224 may be integrally formed. The two reinforcement members 240 are configured to increase the hardness of the first support portion 224, that is, enhance strength of the first air chamber 221.

Specifically, the width of the reinforcement member 240 is greater than or equal to 1 mm and less than or equal to 7 mm. When the wearable electronic device 1000 is worn on the wrist of the user, the first air chamber 221 and the second air chamber 222 are inflated. Because the first air chamber 221 is in contact with the wrist, in a process of inflating the airbag 220, the wristband 200 and an arm apply a clamping force to the airbag 220. The first support portion 224 is located between the first connection portion 223 and the first main body layer 225. The first support portion 224 is provided with the reinforcement member 240. The reinforcement member 240 has sufficient strength to support the first support portion 224 when the first air chamber 221 changes in shape, to prevent the first support portion 224 from shifting in a direction away from the first connection portion 223 during shape change, prevent the first air chamber 221 from shifting from a gap between the first wristband 200a and the wrist of the user, that is, protruding from a side part in a width direction of the first wristband 200a and shifting from a pulse position, and avoid affecting measurement accuracy of the wearable electronic device 1000 while ensuring accuracy of collected test data.

In another embodiment, there is one reinforcement member 240. The reinforcement member 240 is disposed on the first support portion in an extension direction of the first support portion 224. It may be understood that the reinforcement member 240 is of an annular structure.

It should be noted that, during inflation of the airbag 220, the first support portion 224 and the second support portion 228 move away from each other in a thickness direction of the first wristband 200a. In this embodiment, the reinforcement member 240 is rectangular, and contours of two end parts of the reinforcement member 240 in the length direction (the X-axis direction shown in the figure) of the first air chamber 221 are provided with chamfers. In another embodiment, as shown in FIG. 8b, FIG. 8b is a simplified cross-sectional view of the first air chamber in FIG. 2 according to a second embodiment. The reinforcement member 240 is rectangular, and two end parts of the reinforcement member 240 are semi-circular arc contours.

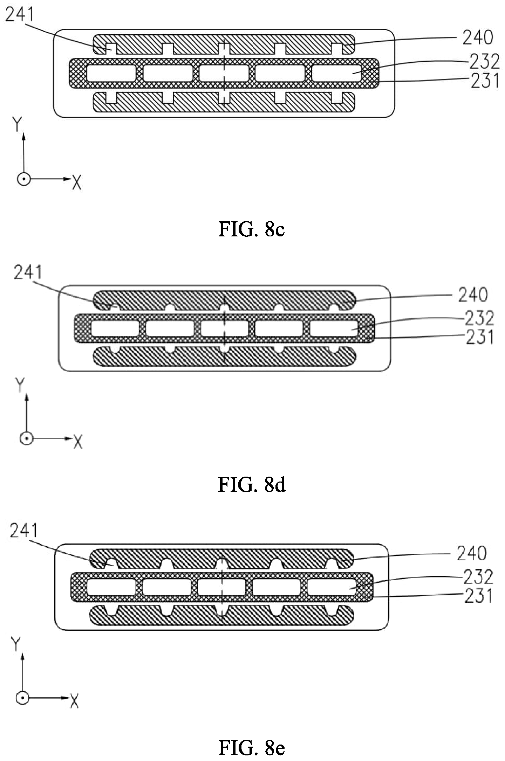

In some embodiments, the reinforcement member 240 is of a hollow structure, to reduce bending stress of the reinforcement member 240. Refer to FIG. 8c and FIG. 8d. FIG. 8c is a simplified cross-sectional view of the first air chamber in FIG. 2 according to a third embodiment, and FIG. 8d is a simplified cross-sectional view of the first air chamber in FIG. 2 according to a fourth embodiment. The reinforcement member 240 is provided with a plurality of hollow portions 241. Each hollow portion 241 penetrates the reinforcement member 240 in a thickness direction of the first air chamber 221 (the reinforcement member 240). In this embodiment, the hollow portion may be a hole or a notch.

In an implementation, the hollow portion 241 penetrates a side edge in the width direction of the reinforcement member 240. In other words, the hollow portion 241 is of a notch structure, and the hollow portion 241 penetrates an edge of the reinforcement member 240, to improve flexibility of the reinforcement member 240, and prevent the first support portion 224 from being wrinkled due to excessive stress of the reinforcement member 240 in a process of expanding the first air chamber 221.

In this embodiment, the plurality of hollow portions 241 are spaced in the length direction (the X-axis direction shown in the figure) of the first air chamber 221. Spacings between every two hollow portions 241 are the same. In another embodiment, spacings between every two hollow portions 241 may alternatively be different. The spacings between every two hollow portions 241 are the same, to ensure stress uniformity of the reinforcement member 240, so that support uniformity of the first support portion is ensured, and uneven local stress affecting detection effect is avoided.

In this embodiment, the two reinforcement members 240 are symmetrical, and the hollow portions 241 of the two reinforcement members 240 are symmetrical relative to a width direction of the connection region 231. In another embodiment, the reinforcement members 240 on both sides in the width direction of the connection region 231 may alternatively be asymmetric. The two reinforcement members 240 are symmetrically disposed, to ensure uniformity of shape change of the first air chamber 221.

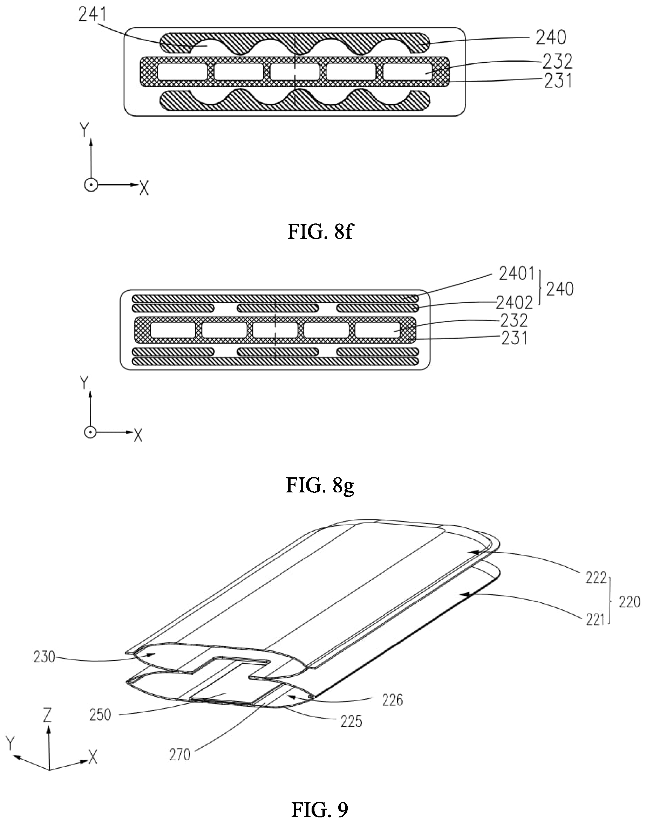

A shape of the hollow portion 241 may be rectangular (as shown in FIG. 8c), a semi-circular arc (as shown in FIG. 8d), arcuate (as shown in FIG. 8e), or wavy (as shown in FIG. 8f). The hollow portion 241 is configured to reduce the bending stress of the reinforcement member 240, so that flexibility of the reinforcement member 240 is improved, and limiting shape change of the airbag 220 during inflation due to the excessive stress of the reinforcement member 240 is avoided.

In an embodiment, in the width direction of the connection region 231, each hollow portion 241 is opposite to a central region of each vent hole 232. In an implementation, the hollow portion and the vent hole 232 are in a regular shape, and a center line of the hollow portion coincides with a center line (a dashed line in the figure) of the vent hole. In another embodiment, in the width direction of the connection region 231, a center line of each hollow portion may alternatively be disposed offset from a center line of each vent hole 232.

In an embodiment, an opening of the hollow portion 241 is disposed to face the connection region 231. In the width direction of the connection region 231, the opening of each hollow portion 241 is opposite to the central region of each vent hole 232. After the airbag is inflated and expands, main body layers around the vent hole 232 changes in shape, some main body layers around the central area of the vent hole 232 change in shape greatly toward an opening direction of the hollow portion 241, and the opening of the hollow portion 241 can exactly provide sufficient shape change space. In another embodiment, the opening of the hollow portion 241 may be disposed opposite to the connection region 231.

In an embodiment, the reinforcement member 240 may be formed by a combination of a plurality of strip bodies, as shown in FIG. 8g. Specifically, the reinforcement member 240 includes a first reinforcement body 2401 and a plurality of second reinforcement bodies 2402. The first reinforcement body 2401 and the second reinforcement body 2402 are spaced in a width direction of the first air chamber 221. The plurality of second reinforcement bodies 2402 are spaced in the length direction of the first air chamber 221. A length of the first reinforcement body 2401 is greater than a length of the second reinforcement body. In this embodiment, three second reinforcement bodies 2402 are disposed, a sum of lengths of the three second reinforcement bodies 2402 and spacings between every two second reinforcement bodies 2402 is equal to the length of the first reinforcement body 2401. The second reinforcement body 2402 is located at two sides of the connection region 231 in a width direction of the first reinforcement body 2401 and the connection region 231. The plurality of second reinforcement bodies 2402 of the reinforcement member 240 are spaced, and spacing regions corresponding to some vent holes may provide the shape change space for the some vent holes during shape change.

In an embodiment, there is one reinforcement member 240. Specifically, the reinforcement member 240 may be disposed around the connection region 231, and is stacked on the first support portion 224. In another embodiment, the reinforcement member may alternatively be located only on a side in the width direction of the connection region 231.

A structure design of the double-layer airbag 220 of the wearable electronic device 1000 provided in this embodiment ensures that the airbag 220 in an inflated state generates sufficient pressure on the wrist of the user, to improve accuracy of test data. After the user starts a blood pressure measurement function, air enters the second cavity 230 of the airbag 220 through the air nozzle 260, and enters the first cavity 226 through the vent hole 232, so that the entire airbag 220 is filled with air, and then blood pressure measurement is performed. In the process of inflating the airbag 220, the reinforcement member 240 disposed on the first support portion 224 of the airbag 220 enhances strength of the first support portion 224 of the airbag 220, to enhance the strength of the first air chamber 221. In addition, in a process of inflating the first air chamber 221, a case in which the first air chamber 221 is subject to the clamping force between the first wristband 200a and the wrist of the user, and bulges out and is displaced from the gap between the first wristband 200a and the wrist of the user is avoided, so that the accuracy of the collected test data by the wearable electronic device 1000 is ensured. The reinforcement member 240 penetrates the plurality of hollow portions 241, reducing the bending stress of the reinforcement member 240, so that bendability of the reinforcement member 240 is improved. During inflation and expansion of the first air chamber 221, the reinforcement member 240 bends together with shape change of the first support portion 224, and does not cause a case in which the shape change of the airbag 220 is limited during inflation due to excessive hardness of the reinforcement member 240.

In an embodiment, the airbag 220 may further include a third air chamber (not shown in the figure), and the third air chamber further includes a third cavity. The third air chamber, the first air chamber 221, and the second air chamber 222 are stacked, and the third air chamber is disposed between the first air chamber 221 and the second air chamber 222. A structure of the third air chamber is basically the same as a structure of the first air chamber, including a fifth main body layer (including a third support portion) and a sixth main body layer (including a fourth support portion). A circumferential edge of the fifth main body layer is connected to a circumferential edge of the sixth main body layer to enclose a third cavity. The fifth main body layer is provided with a fourth vent hole. The sixth main body layer is provided with a third vent hole.

In the thickness direction of the airbag, the first air chamber 221, the third air chamber, and the second air chamber 222 are sequentially connected. A connection portion between the first air chamber 221 and the third air chamber is a connection region. A connection portion between the third air chamber and the second air chamber 222 is a connection region. The two connection regions are formed by aligning vent holes of the first air chamber 221, the third air chamber, and the second air chamber 222. For a specific structural relationship, refer to a connection relationship between the first air chamber and the second air chamber. Details are not described herein again. The airbag 220 is filled with air. In other words, the first cavity 226, the second cavity 230, and the third cavity are filled with air, and the first air chamber 221, the second air chamber 222, and the third airbag all change in shape and expand.

In this embodiment, the reinforcement member 240 is located in the first cavity 226, and is stacked on the first support portion 224 of the first air chamber 221. In another embodiment, the reinforcement member may alternatively be located in the third cavity, and is stacked on the third support portion and the fourth support portion of the third air chamber. Alternatively, the reinforcement member 240 may be stacked on both the third support portion and the first support portion 224. In another embodiment, there may be four or more air chambers of the airbag, and the reinforcement member may be adaptively disposed in each air chamber. This is not listed one by one herein.

Refer to FIG. 9. FIG. 9 is a cross-sectional view of a structure of the airbag shown in FIG. 3 from another perspective. The first air chamber 221 is further provided with a sensor 250. The first main body layer 225 is provided with an attachment region 270. When the user wears the wearable electronic device 1000, the attachment region 270 is a region in which the first air chamber 221 is attached to the wrist of the user. The sensor 250 is located in the first cavity 226, and is embedded in the first main body layer 225. The sensor 250 is configured to collect a pulse wave signal from the wrist artery of the user. The sensor 250 has a wire 251. The wire 251 passes through the first cavity 226 and the vent hole 232 of the connection region 231, extends into the second cavity 230, and extends out from the air nozzle 260, to electrically connect the sensor 250 to the circuit board or a recognition component in the body 100, for an electrical connection between the sensor 250 and the body 100. Specifically, the sensor 250 is a pulse wave sensor (including but not limited to a pressure sensor). When using the wearable electronic device 1000, the user starts inflation of the airbag 220 by operating the control interface of the display 110 of the body 100. The second air chamber 222 and the first air chamber 221 of the first wristband 200a are inflated. The expanded first air chamber 221 is attached to the wrist of the user, and the sensor 250 of the first air chamber 221 is under pressure of the first air chamber 221, and closely attached to wrist skin of the user, to collect the pulse wave signal from the wrist artery at which the user wears the wearable electronic device 1000.

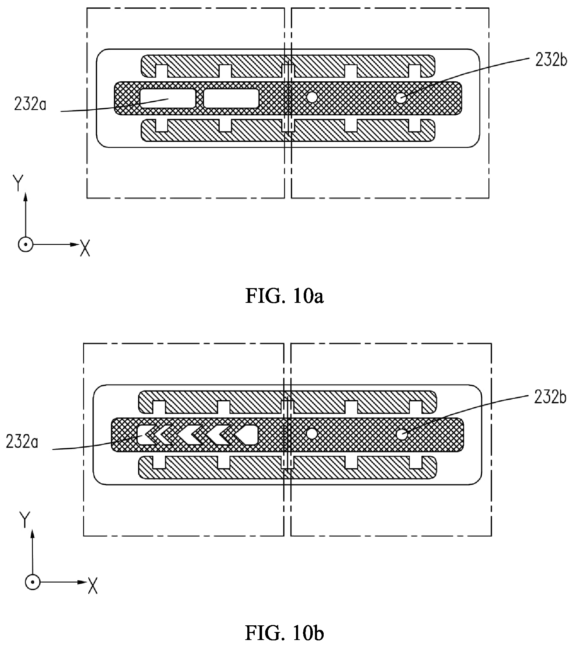

Refer to FIG. 10a and FIG. 10b. FIG. 10a is a simplified diagram of a structure of the airbag in FIG. 2 according to an embodiment, and FIG. 10b is a simplified diagram of a structure of the airbag in FIG. 2 according to another embodiment. The wrist artery of the user includes the ulnar artery and the radial artery. Both the ulnar artery and the radial artery may be detected by the sensor 250 and collected as a pulse signal. When the user wears the wearable electronic device 1000, the first air chamber 221 of the first wristband 200a covers both the ulnar artery and the radial artery of the user. In other words, the first main body layer 225 of the first air chamber 221 covers the ulnar artery and the radial artery of the user. In a length direction of the airbag, the connection region 231 is divided into a first part and a second part, and the vent hole 232 is divided into a plurality of first sub-holes 232a and a plurality of second sub-holes 232b. The first sub-hole 232a is in the first part, and the second sub-hole 232b is in the second part. A cross-sectional area of the first sub-hole 232a is greater than a cross-sectional area of the second sub-hole 232b. The plurality of first sub-holes 232a are spaced in the length direction of the first air chamber 221. The plurality of first sub-holes 232a have a same shape and a same cross-sectional area. The plurality of second sub-holes 232b are spaced in the length direction of the first air chamber 221. The plurality of second sub-holes 232b have a same shape and a same cross-sectional area. In an embodiment, a shape of the first sub-hole 232a is rectangular, and a shape of the second sub-hole 232b is circular, as shown in FIG. 10a. That the cross-sectional area of the first sub-hole 232a is greater than the cross-sectional area of the second sub-hole 232b may be understood as that a ventilation area of the first sub-hole 232a is greater than a ventilation area of the second sub-hole 232b. In a process in which air flows from the second cavity 230 to the first cavity 226 when the airbag 220 is inflated, a ventilation speed at which air passes through the first sub-hole 232a is higher than a ventilation speed at which air passes through the second sub-hole 232b, so that a region that is of the first air chamber 221 and that corresponds to the first sub-hole 232a expands faster, and detection sensitivity of the airbag relative to a region corresponding to the radial artery is higher. In another implementation, a pulse wave sensor may be disposed in the first air chamber 221.

In this embodiment, the cross-sectional area of the first sub-hole 232a is greater than the cross-sectional area of the second sub-hole 232b, so that an ulnar artery pulse signal can be filtered out while a radial artery pulse signal is accurately detected, thereby avoiding interference of the ulnar artery signal with the radial artery signal. In another embodiment, a shape of the first sub-hole 232a is an irregular shape, and a shape of the second sub-hole 232b is circular, as shown in FIG. 10b. A cross-sectional area of the first sub-hole 232a is greater than a cross-sectional area of the second sub-hole 232b. In another embodiment, the second part of the connection region 231 is not provided with a vent hole, so that the radial artery pulse signal can be detected more accurately and quickly.

The wristband 200 further includes a locking portion 280, configured to fasten the first wristband 200a to the second wristband 200b. In this embodiment, the locking portion 280 includes a buckle 281, a buckle tongue 282, and a buckle hole 283. Specifically, the buckle 281 and the buckle tongue 282 are disposed on the first wristband 200a, and the buckle hole 283 is disposed on the second wristband 200b. Alternatively, the buckle 281 and the buckle tongue 282 are disposed on the second wristband 200b, and the buckle hole 283 is disposed on the first wristband 200a.

In this embodiment, the buckle 281 and the buckle tongue 282 are disposed at one end of the first wristband 200a, and the buckle hole 283 is disposed on the second wristband 200b. The buckle 281 is of a rectangular ring structure, and the buckle 281 includes two opposite ends. One end of the buckle 281 is fastened to one end that is of the first wristband 200a and that is away from the second clamping portion, and the buckle tongue 282 is connected to the other end of the buckle 281. A plurality of buckle holes 283 are spaced on the second wristband 200b in a length direction (the X-axis direction shown in the figure) of the second wristband 200b. The buckle hole 283 penetrates the second wristband 200b in a thickness direction (the Z-axis direction) of the second wristband 200b, and is configured to pass through the buckle tongue 282 of the first wristband 200a. When the user wears the wearable electronic device 1000, the first wristband 200a and the second wristband 200b encircle the wrist of the user, and the buckle tongue 282 passes through the buckle hole 283 of the second wristband 200b. The user selects a proper buckle hole 283 from the plurality of buckle holes 283 based on a wrist size, and the buckle tongue 282 passes through the buckle hole 283, to fasten the first wristband 200a to the second wristband 200b.

Embodiments of this application are described in detail above. The principle and implementation of this application are described herein by using specific examples. The descriptions about embodiments are merely provided to help understand the method and core ideas of this application. In addition, a person of ordinary skill in the art makes variations to the specific implementations and the application scope based on the idea of this application. Therefore, the content of this specification shall not be construed as a limitation on this application.

## Claims

1. An airbag, comprising a first air chamber and a second air chamber, wherein the first air chamber and the second air chamber are stacked in a thickness direction of the airbag;
the first air chamber comprises a first main body layer and a second main body layer, the first main body layer and the second main body layer are connected and enclose a first cavity, the second air chamber comprises a third main body layer and a fourth main body layer, the third main body layer and the fourth main body layer are connected and enclose a second cavity, and the first cavity communicates with the second cavity;
the first air chamber and the second air chamber are stacked, the second main body layer and the fourth main body layer are partially connected to form a connection region, a region of the second main body layer and a region of the fourth main body layer that are not connected are non-connection regions, a reinforcement member is stacked on the non-connection region, and the reinforcement member is adjacent to the connection region; and
the airbag is inflated, the first cavity and the second cavity are inflated, the first air chamber and the second air chamber expand in the thickness direction of the airbag, and the reinforcement member drives the non-connection region of the second main body layer away from the fourth main body layer.

2. The airbag according to claim 1, wherein the reinforcement member is provided with a plurality of hollow portions, the plurality of hollow portions are spaced in a length direction of the reinforcement member, and each hollow portion penetrates the reinforcement member in a thickness direction of the first air chamber.

3. The airbag according to claim 2, wherein each hollow portion is provided with an opening, and the opening penetrates a side in a width direction of the reinforcement member.

4. The airbag according to claim 3, wherein the connection region is provided with a plurality of vent holes, the plurality of vent holes connect the first cavity and the second cavity, the plurality of vent holes are spaced in a length direction of the connection region, and in a width direction of the connection region, the plurality of vent holes are in a one-to-one correspondence to the plurality of hollow portions, the opening faces the vent hole, and a middle region of the vent hole is opposite to the opening of the hollow portion.

5. The airbag according to claim 1, wherein hardness of the reinforcement member is greater than hardness of the first air chamber.

6. The airbag according to any one of claims 1 to 5, wherein in a width direction of the first air chamber, a width of the reinforcement member is less than or equal to a width of the non-connection region.

7. The airbag according to claim 2, wherein spacings between every two hollow portions are the same.

8. The airbag according to claim 1, wherein the connection region is provided with a plurality of vent holes, the plurality of vent holes connect the first cavity and the second cavity, and the connection region is divided into a first part and a second part; at least a part of the plurality of vent holes are located in the first part, or a part of the plurality of vent holes are located in the first part, and the other part is located in the second part; and a quantity of vent holes in the first part is the same as a quantity of vent holes in the second part, and a cross-sectional area of the vent hole in the first part is greater than a cross-sectional area of the vent hole in the second part.

9. The airbag according to any one of claims 1 to 5, wherein two end parts of the reinforcement member in a length direction of the first air chamber have a chamfer contour or a semi-circular arc contour.

10. The airbag according to any one of claims 1 to 5, wherein the reinforcement member is fastened to the second main body layer by using a pressing process.

11. The airbag according to any one of claims 1 to 5, wherein the airbag comprises a third air chamber, the third air chamber is stacked between the first air chamber and the second air chamber, the third air chamber comprises a fifth main body layer and a sixth main body layer, the fifth main body layer and the sixth main body layer are respectively partially connected to the second main body layer and the fourth main body layer, and the fifth main body layer and the sixth main body layer have non-connection regions; the reinforcement member is disposed in the non-connection region of the fifth main body layer, and/or the reinforcement member is disposed in the non-connection region of the sixth main body layer.

12. The airbag according to claim 1, wherein the airbag comprises a sensor, an air nozzle, and a wire, the sensor is disposed in the first cavity, the air nozzle is disposed at one end of the second air chamber and connects the outside and the second cavity, and the wire is connected to the sensor and extends out from the air nozzle through the first cavity, the vent hole, and the second cavity.

13. A wristband, comprising a band body and the airbag according to any one of claims 1 to 12, wherein the airbag is disposed on a side of the band body, and the second air chamber is connected to the band body.

14. The wristband according to claim 13, wherein the band body and the airbag are of an integrated structure.

15. A wearable electronic device, comprising a body and the wristband according to claim 13 or 14, wherein the wristband is connected to two opposite ends in a length direction of the body, and the airbag is disposed on an inner side of the wristband.
